# EUROPÄISCHE PATENTSCHRIFT

(11) **EP 3 523 267 B1**
(45) Veröffentlichungstag und Bekanntmachung des Hinweises auf die Patenterteilung: **09.12.2020**
(21) Anmeldenummer: 17777918.8
(22) Anmeldetag: 05.10.2017
(51) Int. Cl.: C07B 57/00, C07C 29/78

(54) **VERFAHREN ZUR ANREICHERUNG VON ENANTIOMEREN AUS EINEM ENANTIOMERENGEMISCH**
METHOD FOR ENRICHMENT OF ENANTIOMERS FROM A MIXTURE OF ENANTIOMERS
PROCÉDÉ D'ENRICHISSEMENT D'ÉNANTIOMÈRES À PARTIR D'UN MÉLANGE D'ÉNANTIOMÈRES

(30) Priorität: 06.10.2016 EP 16192655
(43) Veröffentlichungstag der Anmeldung: 14.08.2019
(73) Patentinhaber: BASF SE, 67056 Ludwigshafen am Rhein (DE)
(72) Erfinder: WLOCH, Sebastian, 67056 Ludwigshafen (DE); HEYDRICH, Gunnar, 67056 Ludwigshafen (DE); RAULS, Matthias, 67056 Ludwigshafen (DE)
(86) Internationale Anmeldenummer: PCT/EP2017/075309
(87) Internationale Veröffentlichungsnummer: WO 2018/065509

(56) Entgegenhaltungen:
- US-A1- 2008 214 877
- HEIKE LORENZ ET AL: "Application of preferential crystallization to resolve racemic compounds in a hybrid process", CHIRALITY., Bd. 18, Nr. 10, 1. Januar 2006 (2006-01-01), Seiten 828-840, XP055341963, US ISSN: 0899-0042, DOI: 10.1002/chir.20327 in der Anmeldung erwähnt

## Beschreibung

Die vorliegende Erfindung betrifft ein Verfahren zur Anreicherung von Enantiomeren aus einem Enantiomerengemisch durch ein fraktionierendes Schmelzkristallisationsverfahren in einer Schmelzkristallisationsvorrichtung. Speziell betrifft die Erfindung ein Verfahren zur Herstellung eines enantiomerenangereicherten chiralen Terpens, insbesondere von D/L-Isopulegol.

Enantiomere weisen mit Ausnahme der optischen Aktivität identische physikalische Eigenschaften auf. Daher lassen sich Mischungen zweier Enantiomere mit den meisten gebräuchlichen verfahrenstechnischen Trennoperationen, wie beispielsweise der Destillation, die auf Unterschieden in den Siedetemperaturen basiert, nicht trennen. Die Trennverfahren für die Enantiomerentrennung oder -anreicherung sind häufig aufwendiger und kostenintensiver. Eine gebräuchliche Methode ist die Trennung auf chromatographischem Wege mittels DC, GC oder HPLC, wobei ein chiraler Komplexbildungspartner die stationäre Phase bildet.

Für viele pharmazeutische Wirkstoffe steht die biochemische Racemattrennung im Vordergrund. Da Enzyme häufig Substrate enantioselektiv umsetzen, werden diese oder entsprechende Mikroorganismen eingesetzt, welche das eine Enantiomer metabolisieren, während das erwünschte Enantiomer erhalten bleibt.

In der Regel einfacher und kostengünstiger können Enantiomere jedoch mit Hilfe der Kristallisation getrennt werden. Eine bekannte Methode der Racemattrennung ist die sogenannte präferentielle bzw. bevorzugte Kristallisation. Dieses Verfahren erlaubt die Auftrennung racemischer Stoffgemische, sofern es sich um sogenannte konglomeratbildende Stoffsysteme handelt, d. h. Stoffsysteme, bei denen die Enantiomere in der festen Phase unmischbar sind.

Wenn das Ausgangsgemisch über die eutektische Zusammensetzung an einem Enantiomer angereichert ist - etwa durch Synthese aus optisch aktiven Ausgangsverbindungen oder durch einen vorgelagerten Chromatographieschritt - gelingt eine enantioselektive Kristallisation. Dabei werden ein Kristallisat des angereicherten Enantiomers und eine Mutterlauge mit der eutektischen Zusammensetzung der Enantiomere erhalten.

So offenbart die WO 2007/023109 ein Verfahren zur Herstellung von enantio- und/oder diastereomerenangereichertem Isopulegol durch Schmelzkristallisation. Das Verfahren gestattet eine Enantiomerenanreicherung unter einfachen apparativen und prozesstechnischen Bedingungen in Abwesenheit von Lösungsmitteln und ohne Zusatz weiterer Hilfsstoffe, wie beispielsweise Kristallisationskeimen.

Beim Schmelzkristallisationsverfahren wird die flüssige Schmelze des zu trennenden Gemischs in einen Kristallisationsapparat gefüllt. An gekühlten Flächen, die unter die Schmelztemperatur des Ausgangsgemisches gekühlt werden, bildet sich eine Kristallschicht mit dem gewünschten Zielprodukt. Unerwünschte Nebenprodukte verbleiben im Wesentlichen in der Mutterschmelze. Die Mutterschmelze wird abgelassen, wodurch eine erste Reinigung erzielt wird. Eine weitere Reinigung kann durch das sogenannte Schwitzen erfolgen; dabei wird über die Kühlflächen die Temperatur der Kristallschicht bis knapp unter die Schmelzpunkttemperatur erhöht. Ein Teil der Kristallschicht an der Grenzfläche zur verbliebenen Mutterschmelze wird dadurch angeschmolzen und verdrängt die verunreinigte Mutterschmelze. Zudem wird hierbei im Kristall eingeschlossene Mutterlauge freigesetzt. Die geschmolzene Schwitzfraktion wird abgelassen; hierdurch wird eine weitere Reinigung des verbliebenen Kristallisats erreicht. Nach Abschluss des Schwitzens wird die verbliebene Kristallschicht abgeschmolzen. Der Umschaltzeitpunkt vom Schwitzen zum Aufschmelzen ist dabei von erheblicher Bedeutung. Wird zu kurz geschwitzt, wird die gewünschte Reinheit des aufgeschmolzenen Kristallisats nicht erreicht. Wird zu lang geschwitzt, wird zwar die gewünschte Reinheit erreicht, es gelangt aber eine unnötig große Menge des reinen Zielprodukts in die Schwitzfraktion. Eine aktive Steuerung des Umschaltzeitpunktes ist umso bedeutender, da Schmelzkristallisationsläufe trotz unveränderter Betriebsparameter nie völlig reproduzierbar verlaufen, insbesondere, wenn hohe Reinheiten des Zielprodukts angestrebt werden.

In der EP 1 924 540 A1 wird ein Verfahren zur Racemattrennung bei verbindungsbildenden Systemen beschrieben, bei dem zunächst aus einer racemischen Mischung eine mit einem Enantiomer angereicherte Fraktion, z. B. mittels Chromatographie, erzeugt wird, um diese in einem zweiten Schritt mit dem gewünschten Enantiomer zu impfen und durch bevorzugte Kristallisation aus einem Lösungsmittel anzureichern. Die Überwachung der einzelnen Verfahrensschritte kann unter anderem durch polarimetrische Messungen, vorzugsweise in Kombination mit anderen Maßnahmen, wie beispielsweise einer Dichtemessung, erfolgen. Durch diese Überwachung soll beispielsweise festgestellt werden können, zu welchem Zeitpunkt die Kristallisation des gewünschten Enantiomers abgebrochen werden muss. Konkret beschrieben wird die Kristallisation von Mandelsäure aus einer wässrigen Lösung, wobei die Zusammensetzung der Lösung durch kombinierte Online-Polarimetrie und Online-Dichtemessung überwacht wurde.

Lorenz, H., Polenske, D. und Seidel-Morgenstern, A., Chirality, 18: 828- 840 (2006) beschreiben, ähnlich wie in EP 1 924 540 A1, die Anwendung einer kombinierten Online-Polarimetrie und Online-Dichtemessung zur Überwachung einer bevorzugten Kristallisation.

In den beschriebenen Veröffentlichungen dient ein Online-Polarimeter bei der bevorzugten Kristallisation der Überprüfung des korrekten thermodynamischen Phasenbereichs, um die gewünschte Komponente auskristallisieren zu können. Eine Anwendung auf dem Gebiet der Schmelzkristallisation wird nicht erwähnt.

Es wurde nunmehr gefunden, dass bei der Anreicherung eines Enantiomeren aus einem Enantiomerengemisch durch ein fraktionierendes Schmelzkristallisationsverfahren in besonders effizienter Weise eine hohe Anreicherung des gewünschten Enantiomers erreicht wird, indem man mittels eines Polarimeters die optische Drehung zumindest der Schwitzfraktion online bestimmt und mittels wenigstens einer Regeleinheit der Wechsel von Schritt ii) zum Schritt iii) online gesteuert wird. Auf diese Weise gelingt zudem eine sehr gut reproduzierbare Anreicherung bei hoher optischer Reinheit des gewünschten Enantiomers.

Die Erfindung betrifft dementsprechend ein Verfahren zur Anreicherung eines Enantiomers aus einem Enantiomerengemisch durch ein fraktionierendes Schmelzkristallisationsverfahren in einer Schmelzkristallisationsvorrichtung, umfassend:
i) einen Kristallisationsschritt unter Erhalt eines Kristallisats und einer Mutterschmelze, und Abtrennen der Mutterschmelze vom Kristallisat unter Gewinnung einer Mutterschmelzefraktion;
ii) Schwitzen des in Schritt i) erhaltenen Kristallisats unter Gewinnung einer geschmolzenen Schwitzfraktion und eines geschwitzten Kristallisats, und
iii) anschließendes Aufschmelzen des geschwitzten Kristallisats unter Gewinnung einer geschmolzenen Kristallisatfraktion,
wobei man mittels eines Polarimeters die optische Drehung zumindest der Schwitzfraktion online bestimmt und mittels wenigstens einer Regeleinheit der Wechsel von Schritt ii) zum Schritt iii) online gesteuert wird.

Unter "online bestimmen" und "online steuern" versteht man grundsätzlich jeweils eine Online-Verarbeitung, die sowohl die Abarbeitung von Aufgaben mit Interaktion des Benutzers (Dialogverarbeitung) als auch ohne Benutzerdialog (Stapelverarbeitung) über ein gemeinsame Kommunikationsnetzwerk umfassen kann. Insbesondere umfasst die Online-Verarbeitung das Abarbeiten einer Befehlsfolge im Rahmen eines Kommunikationsnetzwerkes mit automatischer Übertragung von Daten, gegebenenfalls Speicherung der Daten (zur späteren Weiterverarbeitung oder Dokumentation) und/oder die sofortige Weiterverarbeitung der Daten.

Unter "online bestimmen" soll im Rahmen der vorliegenden Erfindung verstanden werden, dass die optische Drehung der jeweiligen Fraktion, zumindest aber die optische Drehung der Schwitzfraktion im Verfahrensprozess als Messgröße mittels eines Polarimeters kontinuierlich erfasst wird, vorzugsweise ohne dass eine Probenentnahme aus dem System erfolgt.

Unter "online steuern" wird hier und im Folgenden die Übertragung von Daten und/oder Stellgrößen, hier der optischen Drehung der jeweiligen Fraktion, zwischen Sensoren, hier dem Polarimeter, und Aktoren mittels einer aktiven Verbindung über das gemeinsame Kommunikationsnetzwerk verstanden.

Erfindungssgemäß wird in Schritt (c) der Istwert der optischen Drehung der Schwitzfraktion online bzw. inline bestimmt.

Der Begriff "inline bestimmt" wird im Rahmen der vorliegenden Erfindung synonym mit dem Begriff "online bestimmt" verwendet und bedeutet, dass die optische Drehung im Verfahrensprozess kontinuierlich erfasst wird, wobei vorzugsweise keine Probe aus dem laufenden Prozess entnommen wird.

Bei der vorliegenden Regelaufgabe wird daher speziell mittels eines Polarimeters die die optische Drehung der Schwitzfraktion als Messgröße online bzw. inline erfasst, gegebenenfalls der dazugehörige Wert der Enantiomerenüberschusses (EE-Wert), der z.B. mit Hilfe einer zuvor erstellten Kalibrierkurve online aus der optischen Drehung bestimmt werden kann, online bestimmt und mittels einer Regeleinheit der Wechsel von Schritt ii) zum Schritt iii) online gesteuert.

Grundsätzlich können alle Schmelzkristallisationsvorrichtungen verwendet werden, deren Funktion auf der Bildung von Kristallen auf gekühlten Flächen beruht. Das erfindungsgemäße Verfahren kann als dynamisches oder statisches Verfahren durchgeführt werden oder als Kombination dieser beiden Verfahren. Bevorzugt sind statische Schmelzkristallisationsverfahren. Bei den statischen Verfahren wird die flüssige Phase nur durch freie Konvektion bewegt, während bei den dynamischen Verfahren die Kristallisation bei einer erzwungenen Konvektion der flüssigen Phase durchgeführt wird. Dies kann durch eine erzwungene Strömung in volldurchströmten Wärmeüberträgern oder durch die Aufgabe eines Rieselfilms auf eine gekühlte Wand erfolgen.

Vorzugsweise wird das Schmelzkristallisationsverfahren als Schichtkristallisation, insbesondere als statische Schichtkristallisation auf gekühlten Flächen oder als Fallfilmkristallisation, durchgeführt. Die Schmelzkristallisationsvorrichtung ist vorzugsweise als Fallfilmkristallisator oder als Plattenkristallisator ausgestaltet.

Das erfindungsgemäße Verfahren lässt sich demnach auch in Form einer dynamischen Schichtkristallisation durchführen. Im Rahmen einer bevorzugten Ausführungsform führt man diese Variante in Rohrbündelwärmetauschern durch, wie sie in G. F. Arkenbout, Melt Crystallization Technology, Lancater/PA, Technomic Publ. Co., 1995 (Kap. 6.2) beschrieben sind. Dabei werden Schmelze und Kältemittel z. B. in Form eines Rieselfilms an den Innen- und Außenwänden der Wärmetauscher entlanggeführt. Eine derartige Vorrichtung erlaubt eine erleichterte Abtrennung des erhaltenen Kristallisats von der Mutterschmelze bzw. der erhaltenen Schwitzfraktionen durch einfaches Ablaufen unter Schwerkrafteinfluss und bedarf, außer einer Umwälzpumpe, keiner weiteren Rührorgane.

Die flüssige Schmelze des zu trennenden Gemischs wird in die Schmelzkristallisationsvorrichtung gefüllt, üblicherweise mit einer aus dem Schmelzdiagramm ablesbaren Temperatur oberhalb des Schmelzpunktes des reinen Enantiomers. Zur Durchführung einer dynamischen Schichtkristallisation füllt man die flüssige Schmelze des zu trennenden Gemischs ein und führt es durch Umpumpen durch den gekühlten Rohrbündelwärmetauscher. Bei der erfindungsgemäß bevorzugten statischen Schichtkristallisation wird die flüssige Schmelze des zu trennenden bzw. anzureichernden Enantiomerengemischs in den Kristallisationsapparat eingefüllt, welcher kühlbare Flächen, z. B. in Form gekühlter Platten oder Rohre mit gegebenenfalls aufgesetzten Finnen, aufweist.

Die gekühlten Flächen werden unter die Schmelztemperatur des Ausgangsgemisches gekühlt; nach einer gewissen Unterkühlung bildet sich auf den Flächen eine Kristallschicht mit dem gewünschten Enantiomer. Zur Erzielung eines vorteilhaften Kristallisationsergebnisses wird die Absenkung der Kälteträgertemperatur vorzugsweise so gewählt, dass sich innerhalb eines Zeitraumes von etwa 5 h bis etwa 24 h, bevorzugt innerhalb von etwa 7 h bis etwa 19 h eine Kristallschicht einer Stärke von etwa 1 mm bis etwa 50 mm, bevorzugt etwa 5 mm bis etwa 30 mm bildet. Die hierfür erforderlichen Kältemitteltemperaturen liegen in der Regel um etwa 2 K bis etwa 32 K, bevorzugt um etwa 4 K bis etwa 15 K unterhalb der jeweiligen Schmelzetemperatur.

Das nicht erwünschte Enantiomer sowie etwaige sonstige Verunreinigungen verbleiben dabei im Wesentlichen in der Mutterschmelze. Die Schmelze wird weiter abgekühlt bis zu einer bestimmten Endtemperatur. Die dann noch verbleibende Mutterschmelze wird abgelassen. Eine gewisse Menge an verunreinigter Mutterschmelze verbleibt an der Kristallschicht und kann teilweise auch in den Kristallen eingeschlossen sein. Zur weiteren Reinigung sieht das erfindungsgemäße Verfahren einen Schwitzschritt vor. Dabei wird über die Kühlflächen die Temperatur der Kristallschicht bis knapp unter die Schmelzpunkttemperatur erhöht. Ein Teil der Kristallschicht an der Grenzfläche zur verbliebenen Mutterschmelze wird dadurch angeschmolzen und verdrängt die verunreinigte Mutterschmelze. Zudem wird die in den Kristallen eingeschlossene Mutterlauge freigesetzt. Weiterhin können Poren in der Kristallschicht geöffnet werden, die noch vereinigte Mutterschmelze enthalten haben. Hierdurch wird eine weitere Reinigung des verbliebenen Kristallisats erreicht. Vorteilhafte Wärmeträgertemperaturen liegen im Bereich von etwa 0,1 K bis etwa 15 K unterhalb des Schmelzpunkts des jeweils zur Kristallisation eingesetzten Enantiomerengemischs. Bei diesem Prozess des "Schwitzens" können je nach Reinheitsanforderungen etwa 1 bis etwa 70 Gew.-%, häufig etwa 20 bis etwa 50 Gew.-% des Kristallisats wieder aufgeschmolzen werden.

Die beim Schwitzen anfallende flüssige Schwitzfraktion wird durch Drainage entfernt. Nach Abschluss des Schwitzens wird die Temperatur über die Reinstoffschmelztemperatur erhöht, und die verbliebene Kristallschicht wird abgeschmolzen.

Das erfindungsgemäße Verfahren wird in einer oder mehreren Kristallisationsstufen durchgeführt. Allgemein kann man die Kristallisationsstufen in Reinigungsstufen und Abtriebsstufen einteilen. Zur Erhöhung der Trennwirkung können sich einer Kristallisationsstufe weitere Reinigungs-(Kristallisations-)stufen anschließen, in denen jeweils das Kristallisat der vorhergehenden Stufe kristallisiert wird. Zur Erhöhung der Ausbeute des Verfahrens können sogenannte Abtriebsstufen vorgesehen sein, in denen die flüssige Rückstandsphase Abtriebs-(Kristallisations-)stufen unterzogen wird. Vorzugsweise wird hierbei nach dem Gegenstromprinzip vorgegangen, bei dem die Kristallisatströme den Stufen mit der nächsthöheren Stufennummer und die Kristallisationsrückstandsströme den Stufen mit der nächstniedrigeren Stufennummer zugeführt werden. Die Anzahl der Kristallisationsstufen und damit auch der Reinigungs- und Abtriebsstufen hängt von der Trennaufgabe ab und kann vom Fachmann im Rahmen üblicher Versuche ermittelt werden.

Die drei Fraktionen Mutterschmelze (Rückstand), Schwitzfraktion und aufgeschmolzenes Kristallisat werden in der Regel über einen Verteilerblock in separate Behälter, im Folgenden auch Pufferbehälter, gefüllt. Rückstand und aufgeschmolzenes Kristallisat werden je nach Prozessgestaltung entweder als Abfall oder als Wertprodukt aus dem Prozess ausgeschleust, oder falls mehrere Kristallisationsstufen miteinander kombiniert werden, werden die Fraktionen in weitere Abtriebs- oder Reinigungsstufen geführt. Die Schwitzfraktion hat in der Regel eine sehr ähnliche Zusammensetzung wie die ursprüngliche Feed-Zusammensetzung und wird daher intern recycliert, d. h. im nachfolgenden Lauf zusammen mit frischem Feed dem Kristallisator wieder zugeführt.

Erfindungsgemäß bestimmt man online mittels eines Polarimeters die optische Drehung zumindest der Schwitzfraktion. Hierzu sind Prozesspolarimeter besonders geeignet, die nach dem Prinzip der magneto-optischen Kompensation arbeiten. Konventionelle Polarimeter messen die optische Rotation über einen mechanisch gedrehten Analysator. Das mechanische Drehen unterliegt einem Verschleiß und führt nach und nach zum Verlust der Messpräzision. Das Messprinzip der magneto-optischen Kompensation beruht auf dem Faraday-Effekt und kommt ohne bewegte Teile aus. Ein Glasstab, der den Kern einer Magnetspule bildet, wird proportional zu einem Gleichstrom, der durch die Spule fließt, optisch aktiv. Durch Einstellen der Stromstärke und Polarität wird die optische Rotation der Prozessflüssigkeit kompensiert.

Das Polarimeter kann vorzugsweise in der Ablaufleitung der Schmelzkristallisationsvorrichtung für die in den Schritten i) bis iii) anfallenden Schmelzen angeordnet sein. Alternativ kann das Polarimeter auch in einem Bypass der Ablaufleitung installiert sein. Weiterhin ist es möglich, das Polarimeter anstatt in der Ablaufleitung zum Verteilerblock in der Leitung vom Verteilerblock zum Schwitzbehälter zu installieren. Vorzugsweise ist der Ventilblock jedoch unmittelbar stromabwärts des Polarimeters angeordnet.

Mittels wenigstens einer Regeleinheit wird der Wechsel von Schritt ii) zum Schritt iii) online gesteuert. Die wenigstens eine Regeleinheit ist vorzugsweise mit dem Polarimeter datentechnisch gekoppelt. Insbesondere ist diese Regeleinheit als Computer ausgebildet. Im Allgemeinen steuert die Regeleinheit einen in der Ablaufleitung angeordneten Ventilblock, der mit der Ablaufleitung und mit wenigstens zwei Pufferbehältern zur Aufnahme der anfallenden Schmelzen verbunden ist. Dabei steuert die Regeleinheit die Stellung des Ventils (der Ventile), die den Ablauf der Schmelzkristallisationsvorrichtung zu den Behältern für die in den Schritten i) bis iii) anfallenden Schmelzen bestimmen. Daneben ist die Regeleinheit insbesondere ausgebildet, die Temperatur der Kühlflächen der Schmelzkristallisationsvorrichtung zu steuern. Hierzu steuert die Regelvorrichtung vorzugsweise die Kältemitteltemperatur.

Insbesondere geht man so vor, dass man zur Steuerung des Wechsels von Schritt ii) zu Schritt iii)
(a) einen Sollwert für die optische Drehung der Schwitzfraktion festlegt;
(b) eine maximal zulässige Regeldifferenz des Istwerts der spezifischen optischen Drehung vom Sollwert für die optische Drehung der Schwitzfraktion festlegt;
(c) den Istwert der optischen Drehung der Schwitzfraktion online bzw. inline bestimmt;
(d) frühestens bei Erreichen des Sollwerts und spätestens bei Erreichen der maximal zulässigen Regeldifferenz des Istwerts vom Sollwert die Regeleinheit den Wechsel von Schritt ii) zu Schritt iii) bewirkt.

In einer bevorzugten Ausführungsform beträgt die maximale Abweichung, d.h. die maximal zulässige Regeldifferenz, des Istwerts vom Sollwert nicht mehr als 0,3°, insbesondere nicht mehr als 0,2° und speziell nicht mehr als 0,15° optische Drehung bei einer Länge der Messzelle des Polarimeters von 1 dm, einer Temperatur von 25 °C und einer eingesetzten Wellenlänge von 589 nm. Vorzugsweise weicht der Istwert nicht mehr als 0,12 % vom Sollwert ab. Bei einer mehrstufigen Durchführung des erfindungsgemäßen Verfahrens können in der ersten Stufe höhere Abweichungen vom Sollwert toleriert werden als in der letzten Stufe, der sogenannten Produktstufe. Insbesondere ist die maximal zulässige Regeldifferenz des Istwerts vom Sollwert in der Stufe bzw. den Stufen vor der Produktstufe nicht mehr als 0,3°, insbesondere nicht mehr als 0,2°, wohingegen in der letzten Stufe zweckmäßigerweise die Regeldifferenz des Istwerts vom Sollwert nicht mehr als 0,1°, insbesondere nicht mehr als 0,05° und speziell nicht mehr als 0,025° beträgt, jeweils bei einer Länge der Messzelle des Polarimeters von 1 dm, einer Temperatur von 25 °C und einer eingesetzten Wellenlänge von 589 nm.

Der Sollwert der optischen Drehung richtet sich nach dem gewünschten optischen Reinheitsgrad in % EE (Enantiomerenüberschuss). Typischerweise wird man für die optische Drehung der Schwitzfraktion einen Sollwert wählen, der einem EE-Wert von wenigstens 15 % EE, insbesondere einem EE-Wert von wenigstens 20 % entspricht, und je nach gewünschtem Reinheitsgrad oder, im Falle eines Mehrstufigen Kristallisationsverfahrens, je nach Kristallisationsstufe in der Regel im Bereich von 15 bis 99,99 % EE und insbesondere im Bereich von 20 bis 99,9 % EE liegt. Bei einer mehrstufigen Kristallisation wird in der letzten Kristallisationsstufe der Sollwert der optischen Drehung typischerweise einem EE-Wert im Bereich von 95 bis 99,99 % EE und speziell im Bereich von 98 bis 99,9 % EE entsprechen. Vorzugsweise wird die Regeldifferenz in EE-Einheiten nicht mehr als 2 % EE, insbesondere nicht mehr als 1 % EE und speziell nicht mehr als 0,5 % EE betragen.

In einer geeigneten Ausgestaltung weist die Schmelzkristallisationsvorrichtung
einen Kristallisationsraum mit einer Zuleitung für das Einleiten des zu fraktionierenden, geschmolzenen Enantiomerengemischs in den Kristallisationsraum, einem Temperatursensor zum Messen der Temperatur der Schmelze(n) im Kristallisationsraum und einer Temperiereinrichtung zum Verändern der Temperatur des eingeleiteten geschmolzenen Enantiomerengemischs und/oder des daraus teilweise gebildeten Kristallisats,
eine mit einem Ventil absperrbare und mit dem Kristallisationsraum verbundene Ablaufleitung, in der das Polarimeter angeordnet ist oder mit der das Polarimeter über eine Bypassleitung verbunden ist,
gegebenenfalls einen in der Ablaufleitung angeordneten Temperatursensor zum Messen der Temperatur der Schmelze(n) in der Ablaufleitung;
einen in der Ablaufleitung angeordneten Ventilblock, der mit der Ablaufleitung und mit wenigstens zwei Behältern zur Aufnahme der anfallenden Schmelzen verbunden ist, , und
eine Regeleinheit, die mit dem Temperatursensor, dem Polarimeter, der Temperiereinrichtung und einem Ventilblock datentechnisch gekoppelt ist, und mit welcher die Temperatur der festen und flüssigen Phasen in dem Kristallisationsraum und/oder der Ventilblock regelbar ist,
auf.

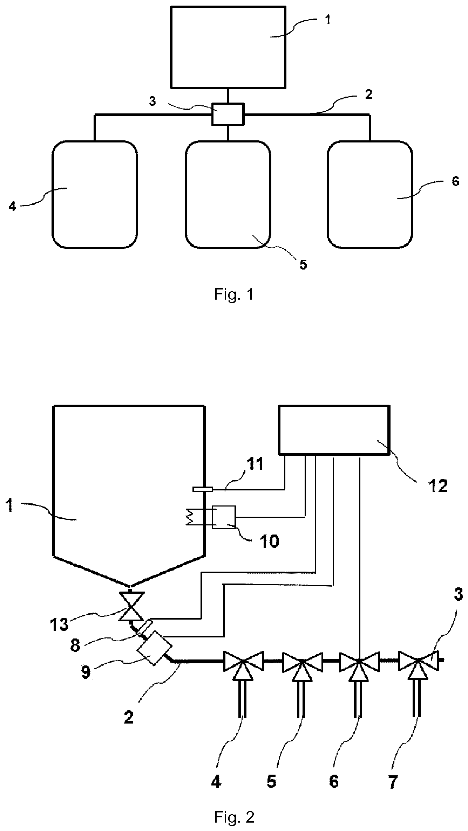

Die Erfindung betrifft auch eine Schmelzkristallisationsvorrichtung, wie sie beispielsweise schematisch in Figur 2 dargestellt ist, die einen Raum für die Kristallisation (1) aufweist, der
eine Zuleitung (7) für das Einleiten des zu fraktionierenden, geschmolzenen Enantiomerengemischs in den Kristallisationsraum (1) aufweist,
einen Temperatursensor (11) zum Messen der Temperatur der Schmelze(n) im Kristallisationsraum (1),
und eine Temperiereinrichtung (10) zum Verändern der Temperatur des eingeleiteten geschmolzenen Enantiomerengemischs sowie des daraus teilweise gebildeten Kristallisats,
wobei die Schmelzkristallisationsvorrichtung weiterhin eine, mit einem Ventil (13) absperrbare, mit dem Kristallisationsraum verbundene Ablaufleitung (2), in der ein Polarimeter (9) angeordnet ist oder mit der das Polarimeter (9) über eine Bypassleitung verbunden ist,
gegebenenfalls einen in der Ablaufleitung (2), vorzugsweise in räumlicher Nähe zum Polarimeter (9), z. B. stromaufwärts des Polarimeters (9), angeordneten Temperatursensor (8) zum Messen der Temperatur der Schmelze in der Ablaufleitung; einen in der Ablaufleitung (2), vorzugsweise stromabwärts des Polarimeters (9), angeordneten Ventilblock (6), der mit der Ablaufleitung (2) und mit wenigstens zwei Pufferbehältern (4), (6) zur Aufnahme der anfallenden Schmelzen und gegebenenfalls einen weiteren Pufferbehälter (5) für Schwitzfraktion verbunden ist, umfasst, und eine Regeleinheit (12) aufweist, die mit den Temperatursensoren (8), (11), dem Polarimeter (9), der Temperiereinrichtung (10) und dem Ventilblock (3) datentechnisch gekoppelt ist, und mit welcher die Temperatur der festen und flüssigen Phasen in dem Kristallisationsraum und/oder der Ventilblock regelbar ist.

Das zu fraktionierende Enantiomerengemisch weist im Allgemeinen keine racemische Zusammensetzung auf. Mit anderen Worten dient ein enantiomerenangereichertes Enantiomerengemisch als Ausgangsmaterial des erfindungsgemäßen Verfahrens. Unter einem enantiomerenangereicherten Gemisch werden solche Gemische verstanden, in denen die beiden Enantiomere nicht im Verhältnis 1 : 1 vorliegen. Der erforderliche Enantiomerenüberschuss hängt von der chemischen Natur des zu fraktionierenden Enantiomerengemisches ab. Für konglomeratbildende Stoffsysteme weist die racemische Zusammensetzung den niedrigsten Schmelzpunkt auf. Eine Reinigung durch Kristallisation ist daher auch bei geringen Enantiomerenüberschüssen im Ausgangsgemisch möglich. Bei verbindungsbildenden Stoffsystemen entspricht die eutektische Zusammensetzung nicht dem Racemat, und eine Reinigung durch Kristallisation ist nur möglich, wenn das Ausgangsgemisch über die eutektische Zusammensetzung an einem Enantiomer angereichert ist. In der Regel wird man zur Kristallisation ein Gemisch der Enantiomere einsetzen, das einen Enantiomerenüberschuss an einem der Enatiomere von wenigstens 10 % EE (Enantiomeric Excess; Enantiomerenüberschuss), insbesondere wenigstens 12,5 % EE und speziell wenigstens 15 % EE, z. B. im Bereich von 10 bis 99,5 % EE, insbesondere im Bereich von 12,5 bis 99,0 % EE, und speziell im Bereich von 15 bis 98,5 % EE aufweist.

Die nach dem erfindungsgemäßen Verfahren zu fraktionierenden Enantiomerengemische unterliegen keinen prinzipiellen Einschränkungen, solange sie ein Eutektikum bilden können. Ein bevorzugter Anwendungsfall betrifft Gemische der Enantiomere eines chiralen Terpens, insbesondere eines chiralen Monoterpens. Als chirale Monoterpene kommen insbesondere Campher und Isopulegol in Betracht. Dabei sind unter Isopulegol die vier möglichen Diastereomere des Isopulegols, nämlich n-Isopulegol, Iso-Isopulegol, Neo-Isopulegol und Neoiso-Isopulegol zu verstehen.

In einer bevorzugten Ausführungsform handelt es sich bei dem chiralen Monoterpen um Isopulegol, insbesondere um D/L-Isopulegol. In der Regel wird man als zu fraktionierendes Enantiomerengemisch ein Gemisch aus D-Isopulegol und L-Isopulegol einsetzen, das einen Enantiomerenüberschuss an einem der Enantiomere von wenigstens 10 % EE (Enantiomeric Excess; Enantiomerenüberschuss), insbesondere wenigstens 12,5 % EE und speziell wenigstens 15 % EE, z. B. im Bereich von 10 bis 99,5 % EE, insbesondere im Bereich von 12,5 bis 99,0 % EE, und speziell im Bereich von 15 bis 98,5 % EE aufweist.

Das erfindungsgemäße Kristallisationsverfahren eignet sich im Rahmen einer bevorzugten Ausführungsform zur Herstellung von enantiomerenangereichertem L-(-)-n-Isopulegol. Synthetisches Menthol wird zumeist über die Zwischenstufe des L-(-)-n-Isopulegols gewonnen, das sich vom Menthol nur durch eine Doppelbindung in der isoPropyl-Seitenkette unterscheidet. Menthol wird hieraus ohne Verlust der Stereospezifität durch Hydrierung erhalten. L-(-)-n-Isopulegol entspricht der Formel (M*) 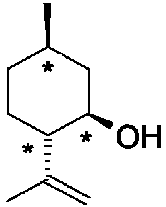 wobei * jeweils ein asymmetrisches Kohlenstoffatom in der dargestellten absoluten Konfiguration bezeichnet.

Die Erfindung betrifft auch ein Verfahren zur Herstellung von Menthol, umfassend
I) die Bereitstellung eines bezüglich des L- oder D-Enantiomers angereicherten Isopulegols durch ein oben beschriebenes Verfahren und
II) Hydrieren des in Schritt I) erhaltenen, bezüglich des L- oder D-Enantiomers angereicherten Isopulegols.

Die Erfindung wird anhand der beigefügten Zeichnungen und der nachfolgenden Beispiele näher veranschaulicht.

Fig. 1 zeigt schematisch eine Schmelzkristallisationsvorrichtung. Die Vorrichtung umfasst einen Kristallisator 1. Die Fraktionen aus dem Kristallisator 1 werden über eine Ablaufleitung 2 und anschließend über einen Ventilblock 3 mit mehreren Ventilen auf die Pufferbehälter für Mutterschmelze 4, Schwitzfraktion 5 und aufgeschmolzenes Kristallisat 6 aufgeteilt.

Fig. 2 zeigt ein Ausführungsbeispiel für die Integration eines Online-Polarimeters in eine statische Schmelzekristallisationsanlage. Der Kristallisator 1 ist über eine mit dem Ventil 13 absperrbare Ablaufleitung 2 mit einem Ventil- bzw. Verteilerblock 3 verbunden. In der Ablaufleitung sitzt eine Temperaturmessung 8 und das Online-Polarimeter 9. Im Ventil- bzw. Verteilerblock wird der Strom aus dem Kristallisator auf den Rückstandsbehälter 4 oder den Schwitzfraktionsbehälter 5 oder den Behälter für aufgeschmolzenes Kristallisat 6 verteilt. Der Feed läuft dem Kristallisator über die Leitung 7 zu. Eine Steuer- und Regeleinheit 12 erhält die Signale der Temperaturmessung und des Online-Polarimeters. Aus gemessener Temperatur und Drehwert wird über die hinterlegte Kalibrierkurve das Enantiomerenverhältnis bestimmt. Bei Erreichen der gewünschten Verhältnisse werden von der Regeleinheit die Ventile umgestellt, um die Ströme in den gewünschten Behälter zu leiten. Weiterhin kann über die Regeleinheit die Temperatur des Kristallisators beeinflusst werden 10, um den EE-Wert beispielsweise der ablaufenden Schwitzfraktion weiter zu beeinflussen. Fig. 2 stellt nur ein Ausführungsbeispiel dar, das Online-Polarimeter kann weiterhin auch in einem Bypass der Ablaufleitung installiert sein. Weiterhin ist es möglich, das Polarimeter anstatt in der Ablaufleitung zum Verteilerblock in der Leitung vom Verteilerblock zum Schwitzbehälter zu installieren.

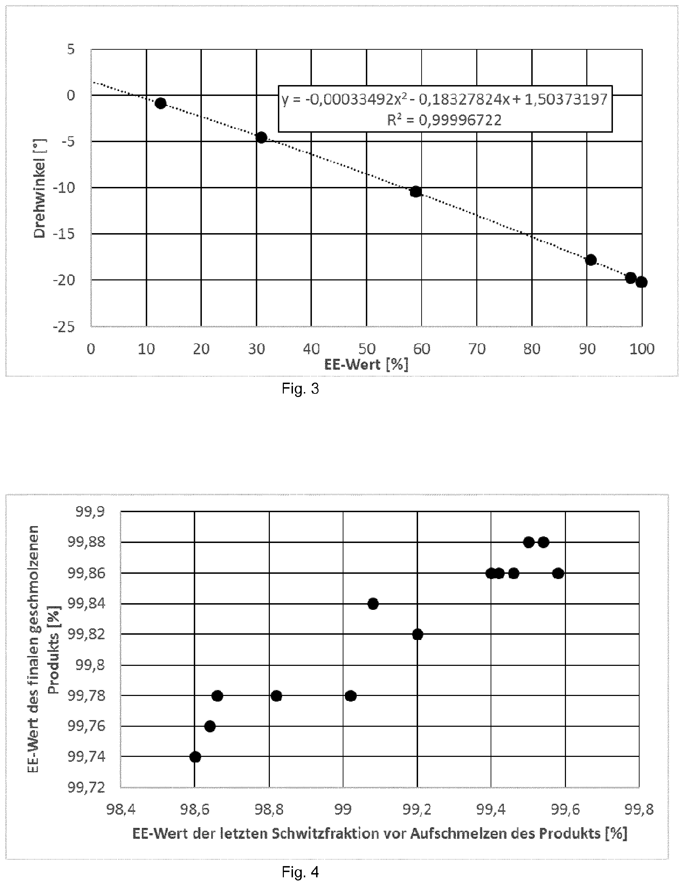

Fig. 3 zeigt eine grafische Auftragung der Drehwerte in Abhängigkeit des EE-Werts für Isopulegol.

Fig. 4 zeigt eine grafische Auftragung der EE-Wert des Produktes in Abhängigkeit des EE-Werts der letzten Schwitzprobe.

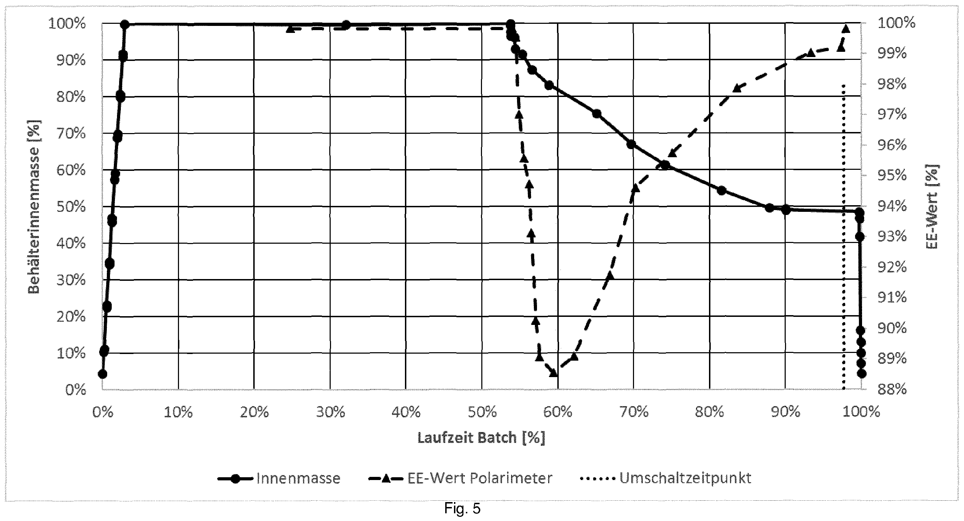

Fig. 5 zeigt den Verlauf des erfindungsgemäßen Verfahrens am Beispiel der letzten Kristallisationsstufe einer mehrstufigen Batch-Kristallisation des Isopulegols in einer Vorrichtung gemäß Fig. 2 mit den Schritten Kristallisation, Schwitzen und Aufschmelzen der Reinfraktion. Aufgetragen ist der zeitliche Verlauf des relativen Anteils der Füllmasse im Kristallisator (= % Innenmasse) und der zeitliche Verlauf des EE-Werts der ablaufenden Isopulegol-Schmelze in EE % über der relativen Zeitdauer des Batches. Die senkrechte gepunktete Linie gibt den Zeitpunkt an, an dem das Ventil zum Schwitzfraktionsbehälter 5 geschlossen wird und ab dem die ablaufende Schmelze im Behälter für aufgeschmolzenes Kristallisat 6 aufgefangen wird.

### Beispiel 1

Drehwertmessungen wurden für das System L- und D-Isopulegol durchgeführt. Ein Perkin Elmer Model 343 Polarimeter mit einer Küvettenlänge von 100 mm und einer Lichtquelle mit einer Wellenlänge von 589 nm wurde genutzt. Alle Messungen fanden bei einer konstanten Temperatur von 25 °C statt. Das Enantiomerenverhältnis von L- und D-Isopulegol wurde in EE-Wert % vorab per Gaschromatographie bestimmt. In Tabelle 1 sind die Messwerte für die Drehwerte in Abhängigkeit des EE-Werts (L-Isopulegol Überschuss) dargestellt.

**Tabelle 1: Messwerte der Drehwertmessungen für unterschiedliche Enantiomerenverhältnisse von L- und D-Isopulegol**

| **EE-Wert [%]** | **Optische Drehung [°]** |
|---|---|
| **12,56** | - 0,82 |
| **30,84** | - 4,522 |
| **58,8** | - 10,42 |
| **90,6** | - 17,77 |
| **97,88** | - 19,66 |
| **99,84** | - 20,17 |

Fig. 3 zeigt eine grafische Auftragung der gemessenen Werte aus Tabelle 1. Die Messwerte können sehr gut mit einem Polynom 2. Ordnung regressiert werden.

### Beispiel 2

Es wurden Betriebsdaten der letzten Kristallisationsstufe einer industriellen Kristallisation von L-Isopulegol ausgewertet. Es wurde eine Schmelzkristallisationsvorrichtung gemäß Fig.1 verwendet. Aus der ablaufenden Schwitzfraktion, welche in den Pufferbehälter 5 geleitet wird, wurden Proben genommen. Die Proben wurden offline mit Hilfe eines Gaschromatographen analysiert. Wenn die Proben einen gewissen minimalen EE-Wert überschritten haben, wurde das Schwitzen abgebrochen und das Kristallisat wurde aufgeschmolzen. Anschließend wurde diese Schmelze als finales Produkt in den Behälter 6 (gemäß Fig. 1 bzw. Fig. 2) geleitet; dabei wurde aus dem Produktstrom ebenfalls eine Probe entnommen und per Gaschromatographie analysiert.

In Tabelle 2 sind die gemessenen EE-Werte der letzten Schwitzfraktionsprobe und des aufgeschmolzenen Produktes aufgelistet.

**Tabelle 2**

| **EE-Wert Wert der letzten Probe der Schwitzfraktion [%]** | **EE-Wert des finalen geschmolzenen Produkts [%]** | **Feed EE-Wert [%]** | **Relative Masse des Produkts in Bezug auf ursprüngliche Feed Masse [%]** |
|---|---|---|---|
| **99,54** | 99,88 | 97,7 | 46 |
| **99,46** | 99,86 | 97,6 | 46 |
| **99,58** | 99,86 | 97,5 | 46 |
| **99,20** | 99,82 | 97,4 | 47 |
| **99,42** | 99,86 | 97,3 | 46 |
| **99,50** | 99,88 | 97,3 | 45 |
| **99,40** | 99,86 | 97,2 | 47 |
| **99,02** | 99,78 | 97,1 | 51 |
| **98,60** | 99,74 | 97,1 | 53 |
| **98,64** | 99,76 | 97,4 | 53 |
| **98,82** | 99,78 | 97,6 | 54 |
| **98,66** | 99,78 | 97,8 | 54 |
| **99,08** | 99,84 | 98,3 | 51 |

In Fig. 4 ist der EE-Wert des Produktes in Abhängigkeit des EE-Werts der letzten Schwitzprobe aufgetragen. Es kann ein deutlicher Zusammenhang zwischen dem EE-Wert der Schwitzprobe und dem EE-Wert des Kristallisats hergestellt werden. Je nach gewünschter Reinheit des Produkts kann ein minimaler EE-Wert der letzten Probe der Schwitzfraktion als Abbruchkriterium festgelegt werden.

Das erfindungsgemäße Verfahren ist auch robust gegenüber schwankenden Ausgangs-EE. So ist in Tabelle 2 auch der Feed EE-Wert aufgelistet, mit dem der Kristallisator ursprünglich befüllt wurde. Trotz schwankender Feed EE-Werte kann der Zusammenhang zwischen dem EE-Wert der Schwitzfraktion und dem EE-Wert des Kristallisats hergestellt werden.

In den Figuren 1 und 2 werden folgende Bezugszeichen verwendet:
- 1: Kristallisationsraum
- 2: Ablaufleitung
- 3: Ventilblock
- 4: Pufferbehälter für anfallende (Mutter-)Schmelze
- 5: Pufferbehälter für Schmelzefraktion
- 6: Pufferbehälter für aufgeschmolzenes Kristallisat
- 7: Zulauf-Leitung
- 8: Temperatursensor
- 9: Polarimeter
- 10: Temperiereinrichtung
- 11: Temperatursensor
- 12: Regeleinheit
- 13: Ventil

## Patentansprüche

1. Verfahren zur Anreicherung eines Enantiomers aus einem Enantiomerengemisch durch ein fraktionierendes Schmelzkristallisationsverfahren in einer Schmelzkristallisationsvorrichtung, umfassend:
i) einen Kristallisationsschritt unter Erhalt eines Kristallisats und einer Mutterschmelze und Abtrennen der Mutterschmelze vom Kristallisat unter Gewinnung einer Mutterschmelzefraktion,
ii) Schwitzen des in Schritt i) erhaltenen Kristallisats unter Gewinnung einer geschmolzenen Schwitzfraktion und eines geschwitzten Kristallisats, und
iii) anschließendes Aufschmelzen des geschwitzten Kristallisats unter Gewinnung einer geschmolzenen Kristallisatfraktion,
wobei man mittels eines Polarimeters die optische Drehung zumindest der Schwitzfraktion online bestimmt und mittels wenigstens einer Regeleinheit der Wechsel von Schritt ii) zu Schritt iii) online gesteuert wird.

2. Verfahren nach Anspruch 1, wobei man zur Steuerung des Wechsels von Schritt ii) zu Schritt iii)
(a) einen Sollwert für die optische Drehung der Schwitzfraktion festlegt;
(b) eine maximal zulässige Regeldifferenz des Istwerts vom Sollwert für die optische Drehung der Schwitzfraktion festlegt;
(c) den Istwert der optischen Drehung der Schwitzfraktion bestimmt;
(d) frühestens bei Erreichen des Sollwerts und spätestens bei Erreichen der maximal zulässigen Regeldifferenz des Istwerts vom Sollwert die Regeleinheit den Wechsel von Schritt ii) zu Schritt iii) bewirkt.

3. Verfahren nach Anspruch 2, wobei die maximal zulässige Regeldifferenz des Istwerts vom Sollwert nicht mehr als 0,3° bei einer Länge der Messzelle des Polarimeters von 1 dm, einer Temperatur von 25 °C und einer eingesetzten Wellenlänge von 589 nm beträgt.

4. Verfahren nach einem der vorhergehenden Ansprüche, wobei die Schmelzkristallisationsvorrichtung eine Ablaufleitung für die in den Schritten i) bis iii) anfallenden Schmelzen aufweist, in der das Polarimeter unmittelbar angeordnet ist oder mit der das Polarimeter über eine Bypassleitung verbunden ist.

5. Verfahren nach Anspruch 4, wobei die Regeleinheit einen in der Ablaufleitung angeordneten Ventilblock steuert, der mit der Ablaufleitung und mit wenigstens zwei Behältern zur Aufnahme der anfallenden Schmelzen verbunden ist.

6. Verfahren nach einem der vorhergehenden Ansprüche, wobei die Schmelzkristallisationsvorrichtung
- einen Kristallisationsraum mit einer Zuleitung für das Einleiten des zu fraktionierenden, geschmolzenen Enantiomerengemischs in den Kristallisationsraum, einem Temperatursensor zum Messen der Temperatur der Schmelze(n) im Kristallisationsraum und einer Temperiereinrichtung zum Verändern der Temperatur des eingeleiteten geschmolzenen Enantiomerengemischs und/oder des daraus teilweise gebildeten Kristallisats;
- eine mit einem Ventil absperrbare und mit dem Kristallisationsraum verbundene Ablaufleitung, in der das Polarimeter angeordnet ist oder mit der das Polarimeter über eine Bypassleitung verbunden ist;
- gegebenenfalls einen in der Ablaufleitung angeordneten Temperatursensor zum Messen der Temperatur der Schmelze(n) in der Ablaufleitung,
- einen in der Ablaufleitung angeordneten Ventilblock, der mit der Ablaufleitung und mit wenigstens zwei Behältern zur Aufnahme der anfallenden Schmelzen verbunden ist, und
- eine Regeleinheit, die mit dem Temperatursensor, dem Polarimeter, der Temperiereinrichtung und einem Ventilblock datentechnisch gekoppelt ist und mit welcher die Temperatur der festen und flüssigen Phasen in dem Kristallisationsraum und/oder der Ventilblock regelbar ist,
aufweist.

7. Verfahren nach einem der vorhergehenden Ansprüche, das wenigstens eines der folgenden Merkmale x1) bis x6) aufweist:
x1) wenigstens eine Regeleinheit ist mit dem Polarimeter datentechnisch gekoppelt;
x2) das Schmelzkristallisationsverfahren wird als Schichtkristallisation durchgeführt;
X3) das Schmelzkristallisationsverfahren wird als statische Schichtkristallisation auf gekühlten Flächen durchgeführt;
x4) das Schmelzkristallisationsverfahren wird als Fallfilmkristallisation durchgeführt;
x5) das zu fraktionierende Enantiomerengemisch weist keine racemische Zusammensetzung auf;
X6) das zu fraktionierende Enantiomerengemisch kann ein racemisches Konglomerat bilden.

8. Verfahren nach einem der vorhergehenden Ansprüche, wobei es sich bei dem zu fraktionierenden Enantiomerengemisch um ein Gemisch der Enantiomere eines chiralen Terpens, insbesondere eines chiralen Monoterpens, handelt.

9. Verfahren nach Anspruch 8, wobei es sich bei dem chiralen Monoterpen um Isopulegol, insbesondere um D/L-Isopulegol handelt.

10. Verfahren nach Anspruch 9, wobei man als fraktionierendes Enantiomerengemisch ein Gemisch aus D-Isopulegol und L-Isopulegol einsetzt, das einen Enantiomerenüberschuss an einem der Enantiomere von wenigstens 10 % EE, insbesondere im Bereich von 12,5 % EE bis 99 % EE, aufweist.

11. Schmelzkristallisationsvorrichtung mit einem Kristallisationsraum (1), der
- eine Zuleitung (7) für das zu fraktionierende, geschmolzene Enantiomerengemisch in den Kristallisationsraum (1),
- einen Temperatursensor (11) zum Messen der Temperatur des geschmolzenen Enantiomerengemischs im Kristallisationsraum (1),
- und eine Temperiereinrichtung (10) zum Verändern der Temperatur des eingeleiteten geschmolzenen Enantiomerengemischs sowie des daraus teilweise gebildeten Kristallisats
aufweist,
wobei die Schmelzkristallisationsvorrichtung weiterhin
- eine, mit einem Ventil (13) absperrbare, mit dem Kristallisationsraum verbundene Ablaufleitung (2), in der ein Polarimeter (9) angeordnet ist oder mit der das Polarimeter (9) über eine Bypassleitung verbunden ist,
- gegebenenfalls einen in der Ablaufleitung (2), vorzugsweise räumlich nah des Polarimeters (9), angeordneten Temperatursensor (8) zum Messen der Temperatur der Schmelze in der Ablaufleitung;
- einen in der Ablaufleitung (2), vorzugsweise stromabwärts des Polarimeters (9), angeordneten Ventilblock (3), der mit der Ablaufleitung (2) und mit wenigstens zwei Pufferbehältern (4), (6) zur Aufnahme der anfallenden Schmelzen verbunden ist,
umfasst, und
eine Regeleinheit (12) aufweist, die mit den Temperatursensoren (8), (11), dem Polarimeter (9), der Temperiereinrichtung (10) und dem Ventilblock (3) datentechnisch gekoppelt ist und mit welcher die Temperatur der festen und flüssigen Phasen in dem Kristallisationsraum und/oder der Ventilblock regelbar ist.

12. Schmelzkristallisationsvorrichtung nach Anspruch 11, die als Fallfilmkristallisator oder als statischer Schichtkristallisator ausgestaltet ist.

13. Verfahren zur Herstellung von einem bezüglich des L- oder D-Enantiomers angereicherten Isopulegol, umfassend ein fraktionierendes Schmelzkristallisationsverfahren eines nicht racemischen Gemischs der D- und L-Enantiomere des Isopulegols nach einem der Ansprüche 1 bis 10.

14. Verfahren nach Anspruch 13, wobei man ein Gemisch aus D-Isopulegol und L-Isopulegol einsetzt, das einen Enantiomerenüberschuss an einem der Enantiomere von wenigstens 10 % EE, insbesondere im Bereich von 12,5 % bis 99 % EE, aufweist.

15. Verfahren zur Herstellung von Menthol, umfassend
I) die Bereitstellung eines bezüglich des L- oder D-Enantiomers angereicherten Isopulegols durch ein Verfahren nach einem der Ansprüche 13 oder 14 und
II) Hydrieren des in Schritt I) erhaltenen, bezüglich des L- oder D-Enantiomers angereicherten Isopulegols.

## Claims

1. A process for enriching an enantiomer from an enantiomer mixture by a fractionating melt crystallization in a melt crystallization apparatus, comprising:
i) a crystallization step to obtain a crystallizate and a mother melt and removal of the mother melt from the crystallizate to afford a mother melt fraction,
ii) sweating of the crystallizate obtained in step i) to afford a molten sweating fraction and a sweated crystallizate, and
iii) subsequent melting of the sweated crystallizate to afford a molten crystallizate fraction,
wherein the optical rotation at least of the sweating fraction is determined online using a polarimeter and the changeover from step ii) to step iii) is controlled online by means of at least one control unit.

2. The process according to claim 1, wherein in order to control the changeover from step ii) to step iii)
(a) a target value for the optical rotation of the sweating fraction is specified;
(b) a maximum permissible control difference of the actual value from the target value for the optical rotation of the sweating fraction is specified;
(c) the actual value for the optical rotation of the sweating fraction is determined;
(d) the control unit effects the changeover from step ii) to step iii) not earlier than upon reaching the target value and not later than upon reaching the maximum permissible control difference of the actual value from the target value.

3. The process according to claim 2, wherein the maximum permissible control difference of the actual value from the target value is not more than 0.3° for a length of the measuring cell of the polarimeter of 1 dm, a temperature of 25°C and an employed wavelength of 589 nm.

4. The process according to any of the preceding claims, wherein the melt crystallization apparatus comprises a discharge conduit for the melts generated in steps i) to iii) in which the polarimeter is located directly or with which the polarimeter is connected via a bypass conduit.

5. The process according to claim 4, wherein the control unit controls a valve block that is located in the discharge conduit and is connected to the discharge conduit and to at least two vessels for receiving the generated melts.

6. The process according to any of the preceding claims, wherein the melt crystallization apparatus has
- a crystallization space having a feed for introduction of the molten enantiomer mixture to be fractionated into the crystallization space, a temperature sensor for measuring the temperature of the melt(s) in the crystallization space and a temperature-control device for varying the temperature of the introduced molten enantiomer mixture and/or of the crystallizate partly formed therefrom;
- a discharge conduit which is closable with a valve and connected to the crystallization space and in which the polarimeter is located or with which the polarimeter is connected via a bypass conduit;
- optionally a temperature sensor located in the discharge conduit for measuring the temperature of the melt(s) in the discharge conduit,
- a valve block that is located in the discharge conduit and is connected to the discharge conduit and to at least two vessels for receiving the generated melts, and
- a control unit which is data-coupled to the temperature sensor, the polarimeter, the temperature-control device and a valve block and with which the temperature of the solid and liquid phases in the crystallization space and/or the valve block is controllable.

7. The process according to any of the preceding claims, having at least one of the following features x1) to x6):
x1) at least one control unit is data-coupled to the polarimeter;
x2) the melt crystallization is performed as a layer crystallization;
x3) the melt crystallization is performed as a static layer crystallization on cooled surfaces;
x4) the melt crystallization is performed as a falling film crystallization;
x5) the enantiomer mixture to be fractionated does not have a racemic composition;
x6) the enantiomer mixture to be fractionated may form a racemic conglomerate.

8. The process according to any of the preceding claims, wherein the enantiomer mixture to be fractionated is a mixture of the enantiomers of a chiral terpene, in particular of a chiral monoterpene.

9. The process according to claim 8, wherein the chiral monoterpene is isopulegol, in particular D/L-isopulegol.

10. The process according to claim 9, wherein a mixture of D-isopulegol and L-isopulegol having an enantiomeric excess of one of the enantiomers of at least 10% EE, in particular in the range from 12.5% EE to 99% EE, is employed as the fractionating enantiomer mixture.

11. A melt crystallization apparatus having a crystallization space (1) which has
- a feed (7) for the molten enantiomer mixture to be fractionated into the crystallization space (1),
- a temperature sensor (11) for measuring the temperature of the molten enantiomer mixture in the crystallization space (1),
- and a temperature-control device (10) for varying the temperature of the introduced molten enantiomer mixture and of the crystallizate partly formed therefrom,
wherein the melt crystallization apparatus further comprises
- a discharge conduit (2) which is closable with a valve (13) and connected to the crystallization space and in which a polarimeter (9) is located or with which the polarimeter (9) is connected via a bypass conduit,
- optionally a temperature sensor (8) located in the discharge conduit (2), preferably in spatial proximity to the polarimeter (9), for measuring the temperature of the melt in the discharge conduit;
- a valve block (3) that is located in the discharge conduit (2), preferably downstream of the polarimeter (9), and is connected to the discharge conduit (2) and to at least two buffer vessels (4), (6) for receiving the generated melts, and
has a control unit (12) which is data-coupled to the temperature sensors (8), (11), the polarimeter (9), the temperature-control device (10) and the valve block (3) and with which the temperature of the solid and liquid phases in the crystallization space and/or the valve block is controllable.

12. The melt crystallization apparatus according to claim 11 which is configured as a falling film crystallizer or as a static layer crystallizer.

13. A process for producing an isopulegol enriched in terms of the L- or D-enantiomer, comprising a fractionating melt crystallization of a non-racemic mixture of the D- and L-enantiomers of isopulegol according to any of claims 1 to 10.

14. The process according to claim 13, wherein a mixture of D-isopulegol and L-isopulegol having an enantiomeric excess of one of the enantiomers of at least 10% EE, in particular in the range from 12.5% to 99% EE, is employed.

15. A process for producing menthol, comprising
I) providing an isopulegol enriched in terms of the L- or D-enantiomer by a process according to either of claims 13 and 14 and
II) hydrogenating the isopulegol enriched in terms of the L- or D-enantiomer obtained in step I).

## Revendications

1. Procédé pour l'enrichissement d'un énantiomère à partir d'un mélange d'énantiomères par un procédé de cristallisation de la masse fondue fractionnant dans un dispositif de cristallisation de la masse fondue, comprenant :
i) une étape de cristallisation avec obtention d'un cristallisât et d'une masse fondue mère et la séparation de la masse fondue mère du cristallisât avec obtention d'une fraction de masse fondue mère,
ii) le ressuage du cristallisât obtenu dans l'étape i) avec obtention d'une fraction ressuée fondue et d'un cristallisât ressué, et
iii) la fusion subséquente du cristallisât ressué avec obtention d'une fraction de cristallisât fondue,
dans lequel on détermine en ligne la rotation optique au moins de la fraction ressuée au moyen d'un polarimètre et on commande en ligne le changement de l'étape ii) à l'étape iii) au moyen d'au moins une unité de réglage.

2. Procédé selon la revendication 1, dans lequel, pour la commande du changement de l'étape ii) à l'étape iii),
(a) on fixe une valeur cible pour la rotation optique de la fraction ressuée ;
(b) on fixe une différence de réglage autorisée maximale de la valeur réelle par rapport à la valeur cible pour la rotation optique de la fraction ressuée ;
(c) on détermine la valeur réelle de la rotation optique de la fraction ressuée ;
(d) au plus tôt lors de l'atteinte de la valeur cible et au plus tard lors de l'atteinte de la différence de réglage autorisée maximale de la valeur réelle par rapport à la valeur cible, l'unité de réglage actionne le changement de l'étape ii) à l'étape iii).

3. Procédé selon la revendication 2, la différence de réglage autorisée maximale de la valeur réelle par rapport à la valeur cible n'étant pas de plus de 0,3° pour une longueur de la cellule de mesure du polarimètre de 1 dm, une température de 25 °C et une longueur d'onde utilisée de 589 nm.

4. Procédé selon l'une quelconque des revendications précédentes, le dispositif de cristallisation de la masse fondue présentant une conduite d'évacuation pour les masses fondues produites dans les étapes i) à iii), dans laquelle le polarimètre est directement disposé ou avec laquelle le polarimètre est relié par l'intermédiaire d'une conduite de by-pass.

5. Procédé selon la revendication 4, l'unité de réglage commandant un bloc de soupapes disposé dans la conduite d'évacuation, qui est relié à la conduite d'évacuation et à au moins deux récipients pour la réception des masses fondues produites.

6. Procédé selon l'une quelconque des revendications précédentes, le dispositif de cristallisation de la masse fondue présentant
- un espace de cristallisation comportant une conduite pour l'introduction du mélange fondu d'énantiomères devant être fractionné dans l'espace de cristallisation, un capteur de température pour la mesure de la température de la masse fondue ou des masses fondues dans l'espace de cristallisation et un dispositif de tempérage pour la modification de la température du mélange fondu d'énantiomères introduit et/ou du cristallisat partiellement formé ;
- une conduite d'évacuation obturable avec une soupape et reliée à l'espace de cristallisation, dans laquelle le polarimètre est disposé ou avec laquelle le polarimètre est relié par l'intermédiaire d'une conduite de by-pass ;
- éventuellement un capteur de température disposé dans la conduite d'évacuation pour la mesure de la température de la masse fondue ou des masses fondues dans la conduite d'évacuation,
- un bloc de soupapes disposé dans la conduite d'évacuation, qui est relié à la conduite d'évacuation et à au moins deux récipients pour la réception des masses fondues produites, et
- une unité de réglage qui est couplée par une technique de données au capteur de température, au polarimètre, au dispositif de tempérage et à un bloc de soupapes et avec laquelle la température des phases solides et liquides dans l'espace de cristallisation et/ou le bloc de soupapes sont réglables.

7. Procédé selon l'une quelconque des revendications précédentes, qui présente au moins l'une des caractéristiques suivantes x1) à x6) :
x1) au moins une unité de réglage est couplée par une technique de données avec le polarimètre ;
x2) le procédé de cristallisation de la masse fondue est mis en œuvre en tant que cristallisation en couche ;
X3) le procédé de cristallisation de la masse fondue est mis en œuvre en tant que cristallisation en couche statique sur des surfaces refroidies ;
x4) le procédé de cristallisation de la masse fondue est mis en œuvre en tant que cristallisation par film tombant ;
x5) le mélange d'énantiomères devant être fractionné ne présente pas de composition racémique ;
X6) le mélange d'énantiomères devant être fractionné peut former un conglomérat racémique.

8. Procédé selon l'une quelconque des revendications précédentes, le mélange d'énantiomères devant être fractionné étant un mélange des énantiomères d'un terpène chiral, en particulier d'un monoterpène chiral.

9. Procédé selon la revendication 8, le monoterpène chiral étant l'isopulégol, en particulier le D/L-isopulégol.

10. Procédé selon la revendication 9, dans lequel on utilise, en tant que mélange d'énantiomères à fractionner, un mélange de D-isopulégol et de L-isopulégol, qui présente un excès énantiomérique de l'un des énantiomères d'au moins 10 % ee, en particulier dans la plage de 12,5 % ee à 99 % ee.

11. Dispositif de cristallisation de la masse fondue comportant un espace de cristallisation (1), qui présente
- une conduite (7) pour le mélange fondu d'énantiomères devant être fractionné dans l'espace de cristallisation (1),
- un capteur de température (11) pour la mesure de la température du mélange fondu d'énantiomères dans l'espace de cristallisation (1),
- et un dispositif de tempérage (10) pour la modification de la température du mélange fondu d'énantiomères introduit ainsi que du cristallisat partiellement formé à partir de celui-ci,
le dispositif de cristallisation de la masse fondue comprenant en outre
- une conduite d'évacuation (2), obturable avec une soupape (13), reliée à l'espace de cristallisation, dans laquelle un polarimètre (9) est disposé ou avec laquelle le polarimètre (9) est relié par l'intermédiaire d'une conduite de by-pass,
- éventuellement un capteur de température (8) disposé dans la conduite d'évacuation (2), de préférence à proximité du polarimètre (9), pour la mesure de la température de la masse fondue dans la conduite d'évacuation ;
- un bloc de soupapes (3) disposé dans la conduite d'évacuation (2), de préférence en aval du polarimètre (9), qui est relié à la conduite d'évacuation (2) et à au moins deux récipients tampon (4), (6) pour la réception des masses fondues produites,
et
présentant une unité de réglage (12), qui est couplée par une technique de données avec les capteurs de température (8), (11), le polarimètre (9), le dispositif de tempérage (10) et le bloc de soupapes (3) et avec laquelle la température des phases solides et liquides dans l'espace de cristallisation et/ou le bloc de soupapes sont réglables.

12. Dispositif de cristallisation de la masse fondue selon la revendication 11, qui est conçu comme cristalliseur par film tombant ou comme cristalliseur en couche statique.

13. Procédé pour la préparation d'un isopulégol enrichi en l'énantiomère L ou D, comprenant un procédé de cristallisation de la masse fondue fractionnant d'un mélange non racémique des énantiomères D et L de l'isopulégol selon l'une quelconque des revendications 1 à 10.

14. Procédé selon la revendication 13, un mélange de D-isopulégol et de L-isopulégol étant utilisé, qui présente un excès énantiomérique de l'un des énantiomères d'au moins 10 % ee, en particulier dans la plage de 12,5 % ee à 99 % ee.

15. Procédé pour la préparation de menthol, comprenant
I) la mise à disposition d'un isopulégol enrichi en l'énantiomère L ou D par un procédé selon l'une quelconque des revendications 13 et 14 et
II) l'hydrogénation de l'isopulégol enrichi en l'énantiomère L ou D obtenu dans l'étape I).
